# EUROPEAN PATENT SPECIFICATION

(11) **EP 3 071 241 B1**
(45) Date of publication and mention of the grant of the patent: **18.09.2019**
(21) Application number: 14802134.8
(22) Date of filing: 24.10.2014
(51) Int. Cl.: A61K 51/02, A61P 35/00, A61P 25/28, A61K 103/00

(54) **ANTICANCER DRUG, COMPRISING A COPPER RADIOISOTOPE**
ANTIKREBSMITTEL MIT EINEM KUPFERRADIOISOTOP
MÉDICAMENT ANTICANCÉREUX CONTENANT UN RADIO-ISOTOPE DU CUIVRE

(30) Priority: 21.11.2013 IT AN20130219
(43) Date of publication of application: 28.09.2016
(73) Proprietor: Valentini, Gianluca, 62100 Macerata (IT)
(72) Inventor: Valentini, Gianluca, 62100 Macerata (IT)
(74) Representative: Premru, Rok
(86) International application number: PCT/IB2014/002261
(87) International publication number: WO 2015/075515

(56) References cited:
- US-A1- 2006 039 859
- European Medicines Agency (emea): "Cuprymina: EPAR - Product Information", , 3 September 2012 (2012-09-03), XP055162251, Retrieved from the Internet: URL:http://www.ema.europa.eu/docs/en_GB/do cument_library/EPAR_-_Product_Information/ human/002136/WC500131689.pdf [retrieved on 2015-01-15]
- GUILLE E ET AL: "TREATMENT OF MICE BEARING A KREBS ASCITIC TUMOR BY MEANS OF A PROTOCOL BASED ON RADIOACTIVE COPPER 64 II. ANALYSIS OF THE TREATMENT", ANTICANCER RESEARCH, vol. 9, no. 4, 1989, pages 947-954, XP008170673, ISSN: 0250-7005
- APELGOT S ET AL: "TREATMENT OF MICE BEARING A KREBS ASCITIC TUMOR BY MEANS OF A PROTOCOL BASED ON RADIOACTIVE COPPER-64 I. THE CANCER ANIMAL A NEW CONCEPT LEADING TO AN EFFECTIVE ANTITUMORAL TREATMENT", ANTICANCER RESEARCH - INTERNATIONAL JOURNAL OF CANCER RESEARCH AND TREATMENT, INTERNATIONAL INSTITUTE OF ANTICANCER RESEARCH, GR, vol. 9, no. 4, 1 January 1989 (1989-01-01) , pages 941-946, XP008170664, ISSN: 0250-7005
- JASON S LEWIS ET AL: "Copper-64-diacetyl-bis(N4-methylthiosemic arbazone): an agent for radiotherapy", PROCEEDINGS OF THE NATIONAL ACADEMY OF SCIENCES, NATIONAL ACADEMY OF SCIENCES, US, vol. 98, no. 3, 30 January 2001 (2001-01-30), pages 1206-1211, XP008110962, ISSN: 0027-8424, DOI: 10.1073/PNAS.98.3.1206
- YUKIE YOSHII ET AL: "Internal radiotherapy with copper-64-diacetyl-bis (-methylthiosemicarbazone) reduces CD133highly tumorigenic cells and metastatic ability of mouse colon carcinoma", NUCLEAR MEDICINE AND BIOLOGY, ELSEVIER, NY, US, vol. 38, no. 2, 24 August 2010 (2010-08-24), pages 151-157, XP028359715, ISSN: 0969-8051, DOI: 10.1016/J.NUCMEDBIO.2010.08.009 [retrieved on 2010-09-02]
- JAMES L. HICKEY ET AL: "Diagnostic Imaging Agents for Alzheimer's Disease: Copper Radiopharmaceuticals that Target A[beta] Plaques", JOURNAL OF THE AMERICAN CHEMICAL SOCIETY, vol. 135, no. 43, 30 October 2013 (2013-10-30), pages 16120-16132, XP055129404, ISSN: 0002-7863, DOI: 10.1021/ja4057807
- HAIYUAN ZHANG ET AL: "Positron Emission Tomography of Human Hepatocellular Carcinoma Xenografts in Mice Using Copper (II)-64 Chloride as a Tracer with Copper (II)-64 Chloride", ACADEMIC RADIOLOGY, RESTON, VA, US, vol. 18, no. 12, 16 August 2011 (2011-08-16), pages 1561-1568, XP028101042, ISSN: 1076-6332, DOI: 10.1016/J.ACRA.2011.08.006 [retrieved on 2011-08-22]
- FANGYU PENG ET AL: "PET of human prostate cancer xenografts in mice with increased uptake of 64CuCl2.", THE JOURNAL OF NUCLEAR MEDICINE, vol. 47, no. 10, 1 October 2006 (2006-10-01), pages 1649-1652, XP055129410, ISSN: 0161-5505
- MCCARTHY D W ET AL: "Efficient Production of High Specific Activity <64>Cu Using A Biomedical Cyclotron", NUCLEAR MEDICINE AND BIOLOGY, ELSEVIER, NY, US, vol. 24, no. 1, 1 January 1997 (1997-01-01), pages 35-43, XP004056509, ISSN: 0969-8051, DOI: 10.1016/S0969-8051(96)00157-6

## Description

The object of the present invention is a drug for the direct treatment of tumours and for the diagnosis and treatment of the Alzheimer's disease as defined by the claims.

The invention falls within the field of anti-cancer drugs using the radioactive properties of certain chemical elements, capable of acting as radionuclides in the form of radiotracers or radiopharmaceuticals, in order to diagnose various pathologies or destroy cancer cells.

In particular, the subject drug uses the radionuclidic properties of copper-64 isotope, for a direct use as copper salt in the treatment of cancer forms and in the diagnosis and treatment of the Alzheimer's disease.

In the field of nuclear medicine, the last 20 years have seen a strong development in the use of the radionuclides, starting from technetium-99m isotope, which represents the most used tracer in SPECT (Single Photon Emission Computed Tomography) diagnostics.

Other radionuclides widely used are iodine-131, especially in the thyroid tumour therapy, and the fluorodeoxyglucose (FDG) in PET (Positron Emission Tomography) diagnostics, which currently represents the most used methodology within the medical-nuclear sphere for the staging and control of oncological therapeutic effects.

In the last two decades the role of copper in the body metabolism and its close relation with the progress of tumoural growth has been widely described.

In fact, copper has a substantial role in the angiogenesis mechanism and in the DNA replication, besides the progress mechanisms of other physiopathologic processes, such as the atherosclerotic plaques, the amyloid plaques (the accumulation whereof among brain neurons is cause of the onset of the Alzheimer's disease) and the Lewy bodies (responsible for the Parkinson's disease).

Since the 60s, researchers such as Cartwright and Wintrobe have shown the high concentration of copper in various organs such as liver, brain, kidneys and pancreas.

Such capability of copper to represent a basic element in the development of various metabolic processes, led the first nuclear medicine researchers to introduce a radioactive isotope thereof (in particular, copper-64) in the study of metabolism: but such copper radionuclide (hereinafter identified with its chemical name "Cu", whereas the relative radioactive isotope shall be identified as "64Cu") had a development more on the aspect of scientific research than of medical application.

The Washington University of Saint Louise has introduced the 64Cu-ATSM in the study of the tumoural necrotic areas; more recently, scientific articles on the 64Cu-Dotatate for endocrine cancer and on the 64Cu-Trastuzumab for breast cancer have been published.

Guillé E. and Apelgot S. "Treatment of mice bearing a Krebs ascitic tumor by means of a protocol based on radioactive copper (64Cu). II. Analysis of the treatment.", Anticancer Research, 9(4), 1989, pages 947-954, discloses ⁶⁴CuCl₂ for treating Krebs ascitic tumours.

Hickey J. L. et al. : "Diagnostic Imaging Agents for Alzheimer's Disease: Copper Radiopharmaceuticals that Target Aβ Plaques", Journal of the Amercian Chemical Society, 135(43), 2013, pages 16120-16132, discloses 64Cu complexes for diagnostic imaging of Alzheimer's Disease.

However, during these years, it has not been at all clarified the role of the 64Cu in its salt form Cl2 (64CuCl2, copper-64 chloride), aimed to a direct use of the cancer disease or of other pathologies.

It should be underlined that the term "direct use" means the use of the copper 64CuCl2 salt molecule alone, that is without the need of synthesizing a complex molecule, comprising the 64Cu bond to other chemical elements, such as in the case of the already known 64Cu-Dotatate and 64Cu-Trastuzumab.

The only studies on this matter, that is on the direct use of the 64Cu in its simple salt form, can be found in a pre-clinical work by Fangyu Peng, which demonstrates the possibility of its diagnostic (but non therapeutic) use in the prostate cancer. The object of the present invention is to provide a pharmaceutical substance comprising 64Cu in its salt form which, per se and without bond with other complex molecules, may be employed in the treatment of certain pathologies, particularly of solid tumours, neuroendocrine tumours, skin tumours, leukaemias and lymphomas.

Another object of the present invention is to provide a pharmaceutical substance which, according to a first dosage, may be employed in the diagnosis of the Alzheimer's disease and, according to a second different dosage, finds use also in the therapeutic treatment of the same disease.

Also disclosed herein is a procedure and suitable means for the production of such pharmaceutical substance. These and other objects, which shall appear clearly hereinafter, are achieved with a drug according to claim 1, usable as therapeutic and diagnostic-therapeutic agent according to claims 4 to 17.

Other objects may also be achieved through the additional features of the dependent claims.

Further features and advantages of the present invention will appear more clearly from the following description of a preferred embodiment thereof, described hereinafter by way of a non-limiting example.

Hereinafter the present description, technical and scientific terms will be used, commonly known by the man skill in the art to which such description is addressed, and sufficiently clear for the invention to be implemented: however, when first mentioning a new element, component or unit of measure the complete name thereof shall be used, subsequently substituted by the relative abbreviation, acronym or brief chemical name when mentioned again. According to the present invention, the chemical composition of the drug consists of copper-64 chloride (64CuCl2), that is copper-64 salt the chemical features thereof are clarified hereinafter.

1 mL of 64CuCl2 solution contains 925 MBq (Mega-Becquerels) of 64Cu at the calibration time of 01h00 a.m. CET (Central European Time) (where such calibration time is defined by the period between the end of the synthesis and the expiry time), corresponding to at least 0.25 µg (micrograms) of 64Cu.

Every vial of the drug 64CuCl2 contains an activity range between 925 MBq and 2,775 MBq at the calibration time, corresponding to an accumulation between 0.25 and 0.75 µg, for a variable volume from 1 to 3 mL.

The minimum specific activity is 3.7 MBq of 64Cu for every µg of 64Cu, at the date and time of expiry.

The 64Cu, with an average life of 12.7 hours, has a Beta+ emission of 17.6% with a maximum energy of 0.66 MeVs (Mega electron Volts), a Beta- emission of 38.5% with a maximum energy of 0.58 MeVs and an electron capture of 43.9%.

Such radionuclide 64Cu decays to stable nickel 64Ni at 61% with a Beta+ emission of 18% or with an electron capture of 43%. It also decays to stable zinc 64Zn with a Beta- emission at 39%.

The possible steps and the process means for obtaining the radionuclide 64Cu, preparatory to the subsequent achievement of the drug 64CuCl2, shall now be described in detail.

The production of 64CuCl2 takes place inside a stainless steel structure with an anti radiation shield: all the inner walls are in fact fully coated with stainless steel, with air tight execution, so as to allow an easy cleaning and decontamination of such structure.

On the front wall there is a suitable zone in plexiglass, which allows the vision of the inside of the structure and ensures the air tight.

Before obtaining the 64CuCl2, takes place the production of 64Cu using a cyclotron, an apparatus situated in a suitably shielded underground bunker and, through a dedicated transport line, in direct connection with the production laboratory.

The material subject to the action of the proton beam by said cyclotron is composed of stable nickel 64Ni, deposited on a gold disk.

Said bombardment action produces the 64Cu, according to the following nuclear reaction responsible for the transmutation: 64Ni (p, n) 64Cu.

The 64Cu thus obtained on the gold disk is transferred, even manually, to the above-mentioned structure, in a special container towards the shielded cell made of lead, where the synthesis module for the drug 64CuCl2 is located, and introduced into the first heating reactor.

On the contrary, the deposit of 64Ni, remaining on the gold disk, is dissolved with hydrochloric acid HCl 6N (6 mL), with subsequent formation of 64NiCl2 (nickel-64 chloride) and 64CuCl2.

The solution thus obtained is purified by making it pass through an ion exchange column, using the following fluxations:
- two fluxations with 5 mL of HCl 6N, for removing nickel impurities,
- one fluxation with 2 mL of HCl 0.5N, for reconditioning the ion exchange column and removing any cobalt present,
- one fluxation with 3 mL of HCl 0.5N, for eluting copper.

The consequent 3 mL of the column (constituting the solution of 64CuCl2) are collected and evaporated to dryness; what remains therefrom is then reconstituted with 3 mL of HCl 0.1N.

The solution of 64CuCl2 thus obtained is finally transferred to the fractionator for the final dispensing thereof in vials, which represents the most suitable container for a drug of this type.

Such drug 64CuCl2 proves to be effective as a therapeutic agent for treating cancer, by triggering specific mechanisms against cancer cells.

Moreover, said drug 64CuCl2 proves to be effective both as a diagnostic agent and as a therapeutic agent against the cells affected by the Alzheimer's disease. With regard to its action as an anti-cancer therapeutic agent, the uptake mechanism of the drug 64CuCl2 by the target cell is presumable to have the following process.

The 64CuCl2 enters the target cell passing the cellular membrane thereof (through methods still not perfectly known, but certainly implying the activation of the serotonin-threonine-kinase enzyme CTR1).

Inside said cell, the 64CuCl2 is used in an equilibrium of secretion and elimination processes of the copper-zinc superoxide dismutase (SOD) and cytochrome oxidase (COX) in the mitochondrions, partly binding to the DNA. In fact, as known from the publication of Maskos and others (Acta Biochim Polonica 1981; 28:183-199), a bond of copper 2+ to the PH-dependent purinic bases takes place.

The 64CuCl2 plays a role of catalyst of the DNA replication reaction, and such role may also have a toxic effect when free radicals hydroxylated with hydrogen peroxide H2O2 or with oxygen anion O- are generated.

Laboratory experimental tests have shown that such drug 64CuCl2, at a certain dosage, has an Auger radioactive effect that is capable of destroying the DNA of the cancer cell, in particular, reducing the volume of the tumoural lesions in solid tumours.

Said dosage is equal to a 1.5 Bq (Becquerel) for each cell, a quantity such that the therapeutic effectiveness of the drug is carried out through the expression of the toxicity thereof only inside the nucleus, achieving a true target therapy. Such toxic effect of the drug 64CuCl2 towards the cancer cell is direct, through the above Auger effect, but also indirect, through the formation of free radicals which are introduced in the cytoplasm and amplify the toxic effect thereof on the DNA (this last feature is already known from studies of 2006 by Buchegger and others, however limited to works on copper intended as a chemical element and not as a radiopharmaceutical).

As said above, the unit dose per cell, required for the drug 64CuCl2 to carry out its anti-cancer therapeutic activity, is equal to 1.5 Bq/cell.

The dose of the drug 64CuCl2 to be administered shall be suitably calculated based on such unit dose of 1.5 Bq/cell, according to the patient, the type of tumour and the extent of the latter.

By way of an example, it has been seen that the average dose for a standard pathology is about 3,700 MBq.

The administration protocol for the drug 64CuCl2 includes a first treatment with the therapeutic dose administered intravenously or arteriously loco-regionally (calculated according to the above-mentioned criteria), to be carried out on a first day and to be repeated on the following day.

Subsequently, the treatment shall be repeated every three months, according to the outcomes of the treatment. Of course the dosage intervals may be modulated and established according to the specific form of cancer treated and to the response of the patient to the drug.

The above described drug 64CuCl2 proved to be effective as a therapeutic agent for the following cancer pathologies: solid tumours (pancreas, prostate, brain, breast, ovary, head-neck, oesophagus, stomach, intestines, colon, liver, lung), neuroendocrine tumours, melanomas, leukaemias and lymphomas.

Laboratory experimental tests, carried out on about one hundred small-sized animals and verified via microPET, have confirmed a reduction in the tumoural mass between 30 and 60% compared to the starting tumoural mass, after only one treatment (according to the administration protocol described above). Compared to other anti-cancer drugs, i.e. compared to chemotherapeutic agents or other radiotherapeutic agents, the drug 64CuCl2 object of this invention allows a number of advantages.

Compared to the drugs used in chemotherapy, said drug 64CuCl2 carries out a therapeutic treatment that is physical and not chemical, through the above Auger effect, by acting only within the nucleus of the target cell.

Moreover, being known the unit dose of drug per cell, it is possible to customise the therapeutic dosage for the specific case, through a dosimetric calculation.

Compared to other types of drugs used in radiotherapy, the drug 64CuCl2 does not need bonds with other complex molecules, as the other currently known radiopharmaceuticals; moreover; it acts directly on the DNA and the relative replication process.

The above is valid with reference to a use of the drug 64CuCl2 as a therapeutic agent in the treatment of cancer forms.

Nonetheless, as mentioned, said drug proved to be effective also against the Alzheimer's disease, both as a diagnostic agent and a therapeutic agent.

The two different uses are based on a different dosage of the drug, being it understood the identical unit dose per cell, equal to 1.5 Bq/cell.

More in particular, it has been seen that through the administration of an average dose of 370 MBq, the drug 64CuCl2 is capable of acting as a radiotracer for the diagnosis of the Alzheimer's disease.

In fact, it is capable of binding to the amyloid plaques, the accumulation whereof within the nerve cells characterises the presence of the pathology, and catalyses its formation process. By crossing the blood-brain barrier, the deposit of the subject drug is suitably identified inside said amyloid plaques, providing a diagnostic feedback beyond all doubts.

With the same uptake - mechanism, but with an average dose administered substantially decupled (equal to 3,700 MBq), the drug 64CuCl2 carries out the function of therapeutic agent against the neuronal cells affected by the Alzheimer's disease.

In fact, the drug, by binding to said amyloid plaques, initially stabilises the growth thereof, to then slower it by braking such "drug-plaque" bonds.

The administration protocol for the drug 64CuCl2, as a therapeutic agent against the neuronal cells affected by the Alzheimer's disease, includes a first treatment with the therapeutic dose administered intravenously or arteriously loco-regionally (calculated according to the above-mentioned criteria), to be carried out on a first day and to be repeated on the following day.

Subsequently, the treatment shall be repeated every three months, according to the outcomes of the treatment. Of course the dosage intervals may be modulated and established according to the specific form of the Alzheimer's disease being treated and to the response of the patient to the drug.

## Claims

1. Drug containing the salt form Cl2 of copper-64 radioisotope 64Cu, **characterised in that**
said drug copper-64 chloride 64CuCl2 is aimed to a direct use in patients, as a substance employable per se and without bond with other complex molecules,
and **in that**
each mL of solution of said drug 64CuCl2 contains 925 MBq of 64Cu at the calibration time of 01h00 a.m. CET, corresponding to at least 0.25 micrograms of said 64Cu.

2. Drug according to claim 1,
**characterised in that**
every vial of said drug 64CuCl2 has a volume between 1 and 3 mL and activity range between 925 and 2,775 MBq, corresponding to an accumulation of 64Cu between at least 0.25 and 0.75 micrograms.

3. Drug according to claim 1,
**characterised in that**
its unit dose for effectiveness is equal to 1.5 Bq for every cell towards which said drug 64CuCl2 is targeted.

4. Drug according to claim 1 for use in the therapeutic treatment of a solid tumour, such as pancreatic, prostate, brain, breast, ovary, head-neck, oesophagus, stomach, intestines, colon, lung cancer.

5. Drug according to claim 1 for use in the therapeutic treatment of a neuroendocrine tumour.

6. Drug according to claim 1 for use in the therapeutic treatment of a melanoma.

7. Drug according to claim 1 for use in the therapeutic treatment of leukaemia or a lymphoma.

8. Drug according to claim 1 for use according to any one of claims 4 to 7, **characterised in that**
it is administered intravenously or arteriously loco-regionally, according to the dosage form of claim 2 and to a dosage customised for the specific form of cancer and for the specific patient, based on the unit dose of claim 3.

9. Drug according to claim 1 for use according to claim 8,
wherein said customised dosage is on the average equal to 3,700 MBq.

10. Drug according to claim 1 for use in the diagnosis of the Alzheimer's disease.

11. Drug according to claim 1 for use according to claim 10,
**characterised in that**
it is administered intravenously or arteriously loco-regionally, according to the dosage form of claim 2 and to a dosage customised for the specific patient, based on the unit dose of claim 3.

12. Drug according to claim 1 for use according to claim 11,
wherein said customised dosage is on the average equal to 370 MBq.

13. Drug according to claim 1 for use in the therapeutic treatment of the Alzheimer's disease.

14. Drug according to claim 1 for use according to claim 13,
**characterised in that**
it is administered intravenously or arteriously loco-regionally, according to the dosage form of claim 2 and to a dosage customised for the specific patient, based on the unit dose of claim 3.

15. Drug according to claim 1 for use according to claim 14,
wherein said customised dosage is on the average equal to 3,700 MBq.

16. Drug according to claim 1 for use according to any one of claims 8 and 15,
**characterised in that**
it is administered a first time on the first day and a second time the next day.

17. Drug according to claim 1 for use according to claim 16,
**characterised in that**
the administration is repeated every three months.

## Patentansprüche

1. Medikament, umfassend das Salz von Cl2 des Radioisotops Kupfer-64 64Cu,
**dadurch gekennzeichnet, dass**
das Medikament Kupfer-64-Chlorid 64CuCl2 für eine direkte Verwendung bei Patienten als eine Substanz bestimmt ist, die per se und ohne Bindung an andere komplexe Moleküle einsetzbar ist,
und dass
jeder ml der Lösung des Medikaments 64CuCl2 925 MBq 64Cu zur Kalibrierungszeit von 01:00 Uhr MEZ umfasst, was mindestens 0,25 Mikrogramm 64Cu entspricht.

2. Medikament nach Anspruch 1,
**dadurch gekennzeichnet, dass**
jede Ampulle des Medikaments 64CuCl2 ein Volumen zwischen 1 und 3 ml und einen Aktivitätsbereich zwischen 925 und 2.775 MBq aufweist, was einer Akkumulation von 64Cu zwischen mindestens 0,25 und 0,75 Mikrogramm entspricht.

3. Medikament nach Anspruch 1,
**dadurch gekennzeichnet, dass**
seine Einheitsdosis zur Wirksamkeit 1,5 Bq für jede Zelle beträgt, gegen die das Medikament 64CuCl2 gerichtet ist.

4. Medikament nach Anspruch 1 zur Verwendung bei der therapeutischen Behandlung eines soliden Tumors, wie Pankreas-, Prostatakrebs, Gehirntumor, Brust-, Eierstock-, Kopf-Hals-Krebs, Speiseröhren-, Magen-, Darm-, Dickdarm-, Lungenkrebs.

5. Medikament nach Anspruch 1 zur Verwendung bei der therapeutischen Behandlung eines neuroendokrinen Tumors.

6. Medikament nach Anspruch 1 zur Verwendung bei der therapeutischen Behandlung eines Melanoms.

7. Medikament nach Anspruch 1 zur Verwendung bei der therapeutischen Behandlung von Leukämie oder eines Lymphoms.

8. Medikament nach Anspruch 1 zur Verwendung nach einem der Ansprüche 4 bis 7,
**dadurch gekennzeichnet, dass**
es gemäß der Dosierungsform von Anspruch 2 und einer Dosierung, die auf die spezifische Krebsform und den spezifischen Patienten zugeschnitten ist, basierend auf der Einheitsdosis von Anspruch 3, intravenös oder arteriell lokoregional verabreicht wird.

9. Medikament nach Anspruch 1 zur Verwendung nach Anspruch 8, wobei die zugeschnittene Dosierung im Durchschnitt 3.700 MBq beträgt.

10. Medikament nach Anspruch 1 zur Verwendung bei der Diagnose der Alzheimer-Erkrankung.

11. Medikament nach Anspruch 1 zur Verwendung nach Anspruch 10,
**dadurch gekennzeichnet, dass**
es gemäß der Dosierungsform von Anspruch 2 und einer Dosierung, die auf den spezifischen Patienten zugeschnitten ist, basierend auf der Einheitsdosis von Anspruch 3, intravenös oder arteriell lokoregional verabreicht wird.

12. Medikament nach Anspruch 1 zur Verwendung nach Anspruch 11, wobei die zugeschnittene Dosierung im Durchschnitt 370 MBq beträgt.

13. Medikament nach Anspruch 1 zur Verwendung bei der therapeutischen Behandlung der Alzheimer-Erkrankung.

14. Medikament nach Anspruch 1 zur Verwendung nach Anspruch 13,
**dadurch gekennzeichnet, dass**
es gemäß der Dosierungsform von Anspruch 2 und einer Dosierung, die auf den spezifischen Patienten zugeschnitten ist, basierend auf der Einheitsdosis von Anspruch 3, intravenös oder arteriell lokoregional verabreicht wird.

15. Medikament nach Anspruch 1 zur Verwendung nach Anspruch 14, wobei die zugeschnittene Dosierung im Durchschnitt 3.700 MBq beträgt.

16. Medikament nach Anspruch 1 zur Verwendung nach einem der Ansprüche 8 und 15,
**dadurch gekennzeichnet, dass**
es zum ersten Mal am ersten Tag und zum zweiten Mal am nächsten Tag verabreicht wird.

17. Medikament nach Anspruch 1 zur Verwendung nach Anspruch 16,
**dadurch gekennzeichnet, dass**
die Verabreichung alle drei Monate wiederholt wird.

## Revendications

1. Médicament contenant la forme saline Cl2 du radio-isotope 64Cu en cuivre 64,
**caractérisé en ce que**
ledit médicament 64CuCl2 en cuivre-64 est destiné à une utilisation directe chez les patients, en tant que substance utilisable en soi et sans liaison avec d'autres molécules complexes,
et **en ce que**
chaque mL de solution dudit médicament 64CuCl2 contient 925 MBq de 64Cu au moment de l'étalonnage de 01h00 am CET, correspondant à au moins 0,25 microgramme dudit 64Cu.

2. Médicament selon la revendication 1,
**caractérisé en ce que**
chaque flacon dudit médicament 64CuCl2 a un volume compris entre 1 et 3 mL et une activité située entre 925 et 2775 MBq, ce qui correspond à une accumulation de 64Cu comprise entre au moins 0,25 et 0,75 microgramme.

3. Médicament selon la revendication 1,
**caractérisé en ce que**
sa dose unitaire d'efficacité est égale à 1,5 Bq pour chaque cellule visée par le médicament 64CuCl2.

4. Médicament selon la revendication 1 à utiliser dans le traitement thérapeutique d'une tumeur solide, telle que le pancréas, la prostate, le cerveau, le sein, les ovaires, la tête, le cou, l'oesophage, l'estomac, les intestins, le colon, le cancer du poumon.

5. Médicament selon la revendication 1 à utiliser dans le traitement thérapeutique d'une tumeur neuroendocrine.

6. Médicament selon la revendication 1 à utiliser dans le traitement thérapeutique d'un mélanome.

7. Médicament selon la revendication 1 à utiliser dans le traitement thérapeutique de la leucémie ou d'un lymphome.

8. Médicament selon la revendication 1 à utiliser selon l'une quelconque des revendications 4 à 7,
**caractérisé en ce que**
il est administré par voie intraveineuse ou artérielle locorégionale, selon la forme posologique de la revendication 2 et selon un dosage personnalisé pour la forme spécifique de cancer et pour le patient spécifique, sur la base de la dose unitaire de la revendication 3.

9. Médicament selon la revendication 1 à utiliser selon la revendication 8, dans lequel ledit dosage personnalisé est en moyenne égal à 3700 MBq.

10. Médicament selon la revendication 1 à utiliser dans le diagnostic de la maladie d'Alzheimer.

11. Médicament selon la revendication 1 à utiliser selon la revendication 10,
**caractérisé en ce que**
il est administré par voie intraveineuse ou artérielle au niveau régional, conformément à la forme posologique de la revendication 2 et à une posologie adaptée au patient spécifique, sur la base de la dose unitaire de la revendication 3.

12. Médicament selon la revendication 1 à utiliser selon la revendication 11, dans lequel ladite dose personnalisée est en moyenne égale à 370 MBq.

13. Médicament selon la revendication 1 à utiliser dans le traitement thérapeutique de la maladie d'Alzheimer.

14. Médicament selon la revendication 1 à utiliser selon la revendication 13,
**caractérisé en ce que**
il est administré par voie intraveineuse ou artérielle locorégionale, selon la forme posologique de la revendication 2 et selon une posologie adaptée au patient spécifique, sur la base de la dose unitaire de la revendication 3.

15. Médicament selon la revendication 1 à utiliser selon la revendication 14, dans lequel ledit dosage personnalisé est en moyenne égal à 3 700 MBq.

16. Médicament selon la revendication 1 à utiliser selon l'une quelconque des revendications 8 et 15,
**caractérisé en ce que**
il est administré une première fois le premier jour et une seconde fois le lendemain.

17. Médicament selon la revendication 1 à utiliser selon la revendication 16,
**caractérisé en ce que**
l'administration est répétée tous les trois mois.
